Europäisches Patentamt

European Patent Office

Office européen des brevets

(11) Veröffentlichungsnummer: **0 570 787 A2**

# EUROPÄISCHE PATENTANMELDUNG

(21) Anmeldenummer: **93107517.0**

(22) Anmeldetag: **08.05.93**

(51) Int. Cl.5: **C12N 15/90**, C12N 15/57, C12N 15/56, C12N 1/21, //(C12N1/21,C12R1:07)

(30) Priorität: **16.05.92 DE 4216246**

(43) Veröffentlichungstag der Anmeldung: **24.11.93 Patentblatt 93/47**

(84) Benannte Vertragsstaaten: **AT BE CH DE DK ES FR GB GR IE IT LI LU NL PT SE**

(71) Anmelder: **Solvay Enzymes GmbH & Co. KG Grosse Drakenburger Strasse 95 D-31582 Nienburg(DE)**

(72) Erfinder: **Vetter, Roman Warneckeweg 1 W-3167 Burgdorf(DE)**
Erfinder: **Wilke, Detlef Landwehr 6 W-3015 Wenningsen(DE)**
Erfinder: **Koeller, Magret Kronsberger Strasse 16 W-3000 Hannover 71(DE)**
Erfinder: **Möller, Bernhard Neuweilerstrasse 12 W-5110 Alsdorf(DE)**
Erfinder: **Amory, Antoine Avenue Bel Air 44 B-1330 Rixensart(DE)**

(74) Vertreter: **Lauer, Dieter, Dr. c/o Solvay Deutschland GmbH, Hans-Böckler-Allee 20 D-30173 Hannover (DE)**

(54) **Einfaches Verfahren zur stabilen chromosomalen Genamplifikation.**

(57) Die Erfindung betrifft ein einfaches Verfahren zur stabilen chromosomalen Genamplifikation von DNA-Sequenzen, die für ein Polypeptid in prokaryotischen Mikroorganismen, insbesondere in Bacillus-Stämmen codieren.

EP 0 570 787 A2

EP 0 570 787 A2

Die Erfindung betrifft ein einfaches Verfahren zur stabilen chromosomalen Genamplifikation von DNA-Sequenzen, die für ein Polypeptid in prokaryotischen Mikroorganismen, insbesondere in Bacillus-Stämmen, codieren.

Mikroorganismen, insbesondere Bacillus-Stämme, finden weitreichende industrielle Anwendung zur mikrobiologischen Herstellung von Polypeptiden, insbesondere zur Herstellung von wichtigen Bacillus-Enzymen wie z.B. Proteasen oder Amylasen etc. Ein wichtiges Ziel in der Optimierung der Herstellung solcher Enzyme besteht darin, die Expression dieser Enzyme durch die Bacilli zu erhöhen. Hierzu kann z.B. die Regulation der Expression optimiert werden oder die genetische Information für das Enzym und dessen Expression im Bacillus vervielfacht werden. Zur Vervielfältigung der genetischen Information wird im Stand der Technik einerseits die Erhöhung der Anzahl von Plasmiden mit der benötigten genetischen Information im Bacillus vorgeschlagen, bzw. andererseits versucht, die Expression für das interessierende Polypeptid durch mehrfachen Einbau der dafür codierenden genetischen Information in die chromosomale DNA des Bacillus zu steigern. Die Erhöhung der Plasmidanzahl ist jedoch aus Stabilitätsgründen für großtechnische Produktionsverfahren der interessierenden Enzyme ungeeignet. Im Stand der Technik wird daher zur Steigerung der Expression bevorzugt der mehrfache Einbau der genetischen Information in die Chromosomen-DNA angestrebt.

Es bestand daher die Aufgabe, ein einfaches, allgemein anwendbares Verfahren bereitzustellen, bei dem die für ein interessierendes Polypeptid, vorzugsweise für ein Enzym codierende DNA-Sequenz, durch stabile, chromosomale Integration zusätzlicher Kopien dieser DNA-Sequenz in das Genom eines Organismus, vorzugsweise eines Bacillus, vervielfacht wird.

Die Aufgabe wird gelöst durch das erfindungsgemäße Verfahren zur Herstellung einer transformierten prokaryotischen Wirtszelle, die die für ein interessierendes Polypeptid codierende DNA-Sequenz wenigstens zweifach chromosomal enthält, welches sich dadurch auszeichnet, daß man eine prokaryotische Wirtszelle, die wenigstens bereits eine für ein interessierendes Polypeptid codierende DNA-Sequenz in ihrer chromosomalen DNA enthält, mit einer zur Transformation der Wirtszelle geeigneten zirkulären DNA aus im wesentlichen

a) einem DNA-Fragment, welches die für ein interessierendes Polypeptid codierende DNA-Sequenz einschließlich der für die Expression und gegebenenfalls für die Sekretion des interessierenden Polypeptids benötigten Sequenzen enthält, und aus

b) wenigstens einem DNA-Fragment mit einer DNA-Sequenz, die für einen selektierbaren Marker codiert, wobei diese zirkuläre DNA keine DNA-Sequenz enthält, die sie zur autonomen Replikation in der Wirtszelle befähigt, transformiert,

anschließend mit Hilfe des selektierbaren Markers solche Zellen, die die verwendete zirkuläre DNA wenigstens einmal chromosomal integriert haben, selektiert und die derart transformierten Zellen isoliert.

Daß die zur Transformation geeignete zirkuläre DNA im wesentlichen das Fragment a) und das Fragment b) enthält, bedeutet im Sinne der Erfindung, daß auch noch kurze DNA-Sequenzen, beispielsweise Linker-Sequenzen, wie sie zur Konstruktion der zirkulären DNA benötigt wurden, enthalten sein können.

Die in Fragment a) enthaltenden für die Expression und gegebenenfalls für die Sekretion des interessierenden Polypeptids benötigten Sequenzen sind die hierfür notwendigen Promotor-, Operator-, Enhancer-, Terminator- und Signalsequenzen und weiterhin gegebenenfalls auch Prä- und Prosequenzen, wie sie beispielsweise zur Sekretion einer Protease notwendig sind.

Als für selektierbare Marker im Fragment b) codierende DNA-Sequenzen können alle allgemein üblichen DNA-Sequenzen eingesetzt werden, die eine Selektion der transformierten Zellen ermöglichen. Derartige allgemein übliche Selektionsverfahren sind beispielsweise Selektionen auf der Grundlage von auxotrophen Mutationen oder vorzugsweise Antibiotikum-Resistenzen der verwendeten Zellen.

Zweckmäßigerweise werden im erfindungsgemäßen Verfahren als Wirtsorganismen Bacilli, vorzugsweise Bacillus alcalophilus, Bacillus licheniformis oder Bacillus subtilis eingesetzt. Zweckmäßige Polypeptide sind z.B. Proteasen, vorzugsweise alkalische und insbesondere hochalkalische Proteasen, sowie $\alpha$-Amylasen, Lipasen oder Cellulasen.

Die Integration der DNA-Sequenz, die für das interessierende Polypeptid codiert, in das Genom der Wirtszelle wird im erfindungsgemäßen Verfahren durch einfache homologe Rekombination erreicht. Hierzu wird eine Wirtszelle, die wenigstens bereits eine DNA-Sequenz für das interessierende Polypeptid und die für die Expression dieses Polypeptids benötigten DNA-Sequenzen enthält, erfindungsgemäß mit einer zirkulären DNA transformiert, die homologe DNA-Sequenzen zur chromosomalen Wirtszellen-DNA aufweist. Hierbei entsprechen diese homologen DNA-Sequenzen gleichzeitig dem DNA-Fragment a), welches die für das interessierende Polypeptid codierende DNA-Sequenz enthält. Erfindungsgemäß ist daher für die homologe Rekombination keine weitere spezielle, zur chromosomalen DNA des Wirtsorganismus homologe Ziel-DNA-Sequenz (Target-DNA-Sequenz) erforderlich, da deren Funktion durch das vorstehende DNA-

2

Fragment a) mit der für ein interessierendes Polypeptid codierenden DNA-Sequenz mit erfüllt wird. Ferner enthält die hier verwendete zirkuläre DNA zumindest eine Marker-DNA-Sequenz, die beispielsweise für eine Antibiotikum-Resistenz codiert und die nach erfolgter Transformation des Wirtsorganismus mit dieser zirkulären DNA zur Selektion von solchen Transformanten dient, in denen mehr als eine für das interessierende Polypeptid codierende DNA-Sequenz enthalten ist. Um sicherzustellen, daß nur solche Transformanten erhalten und selektiert werden, in denen die zirkuläre DNA in die chromosomale DNA des Wirtsorganismus integriert wurde, besitzt die verwendete zirkuläre DNA keinerlei DNA-Sequenzen, die eine autonome Replikation dieser DNA in dem Wirtsorganismus ermöglichen. Eine Weitergabe der zirkulären DNA in Form von Plasmiden ist somit ausgeschlossen. Nach dem erfindungsgemäßen Verfahren enthalten die transformierten Wirtszellen die für das interessierende Polypeptid codierende DNA-Sequenz stabil in ihr Genom integriert.

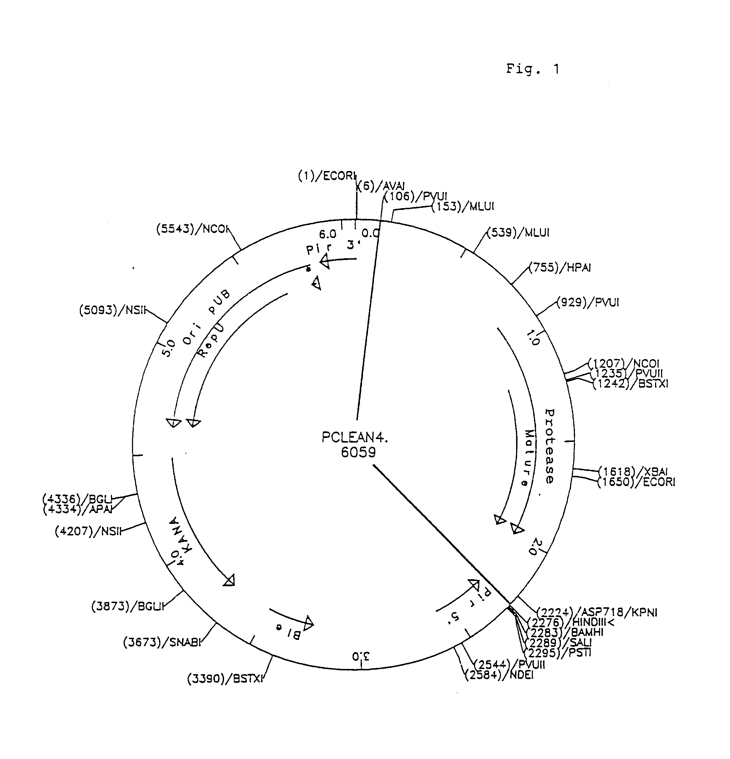

Zur Herstellung der für die Transformation im erfindungsgemäßen Verfahren benötigten zirkulären DNA kann nach an sich üblichen Methoden vorgegangen werden. Hierzu wird zunächst die für das interessierende Polypeptid codierende DNA-Sequenz, sowie die für die Polypeptidexpression und gegebenenfalls Polypeptidsekretion in einem Wirtsorganismus benötigten regulatorischen Sequenzen, Signalsequenzen, Promotorsequenzen, Terminatorsequenzen und die für die Pre- und Proeinheiten des Polypeptids codierenden DNA-Sequenzen aus einem Mikroorganismus isoliert, der an sich das interessierende Polypeptid produziert. Beispielsweise wird aus einem Bakterium ("Donor-Bakterium"), insbesondere aus einer Bacillus-Spezies, die das interessierende Polypeptid, z.B. eine Protease oder Amylase produziert, die chromosomale DNA nach an sich bekannten Methoden isoliert und mit geeigneten Restriktionsendonukleasen partiell hydrolysiert. Die erhaltenen Restriktionsfragmente der Donor-DNA können z.B. durch Gelelektrophorese nach Größe aufgetrennt und die Fragmente gewünschter Größe dann mit einer geeigneten Vektor-DNA, z.B. einer Plasmid-DNA rekombiniert werden. Mit dem in vitro rekombinierten Plasmid (Vektor-DNA + Restriktionsfragmente der Donor-DNA) können Bakterien, vorzugsweise eine Bacillus-Spezies, transformiert werden und die Transformanten nach der bekannten Markereigenschaft der Vektor-DNA (Antibiotikum-Resistenz) selektiert werden. Man sucht unter diesen Transformanten nach solchen Klonen, die das interessierende Polypeptid, z.B. eine Protease oder Amylase, ausscheiden. Aus einem Klon mit der gewünschten Polypeptidaktivität wird schließlich die in diesen Transformanten eingeführte Plasmid-DNA isoliert. Durch erneute Transformation eines Bakteriums kann überprüft werden, ob die Polypeptidaktivität Plasmidgebunden ist, d.h. ob die Polypeptidaktivität mit der Markereigenschaft gekoppelt ist. Das so isolierte Plasmid enthält neben der Vektor-DNA mit bekannten Restriktionsstellen das gewünschte Strukturgen für das interessierende Polypeptid, sowie die für die Polypeptidexpression benötigten regulatorischen Sequenzen, Signalsequenzen, Promotorsequenzen und Terminatorsequenzen und gegebenenfalls die für die Pre- und Proeinheiten des Polypeptids codierenden DNA-Sequenzen. Enthält der Vektor noch weitere, hier aber nicht benötigte DNA-Sequenzen aus dem Donor-Bakterium, so empfiehlt es sich gegebenenfalls, diese nicht benötigten Sequenzen vor der weiteren Verwendung des Vektors zu eliminieren und die Donor-DNA-Sequenz im wesentlichen auf das Strukturgen für das Polypeptid und auf die für dessen Expression und gegebenenfalls Sekretion benötigten DNA-Sequenzen zu reduzieren. Hierzu wird z.B. das Plasmid, welches die zusätzlichen nicht benötigten DNA-Sequenzen umfaßt, mit einer Anzahl verschiedener Restriktionsendonukleasen geschnitten (restringiert), die erhaltenen DNA-Fragmente durch Gelelektrophorese nach Größe getrennt und anhand des gefundenen Bandenmusters eine Restriktionskarte erstellt. Es werden so die Restriktionsstellen, die im Bereich der Donor-DNA-Sequenz angesiedelt sind, ermittelt. Die Kenntnis der Restriktionskarte des Plasmids ermöglicht es nunmehr, aus diesem Plasmid durch Schneiden mit ausgewählten Restriktionsendonukleasen ein DNA-Fragment der Donor-DNA-Sequenz herauszuschneiden, welches im wesentlichen nur noch die benötigten Sequenzen aus dem Donor-Bakterium umfaßt. Durch den Wiedereinbau dieser, in der Größe reduzierten Donor-DNA-Sequenz in einen geeigneten Vektor kann ein neues, Plasmid erhalten werden, das zur Polypeptidexpression befähigt ist und das als Grundlage für die Erzeugung der im erfindungsgemäßen Verfahren verwendeten zirkulären DNA dienen kann. Ein Beispiel für ein solches reduziertes Plasmid mit der Bezeichnung pCLEAN4 gibt die Restriktionskarte der Figur 1 wieder. In diesem Plasmid ist das interessierende Polypeptid eine hochalkalische Protease aus einem Bacillus alcalophilus.

Zur Erzeugung der im erfindungsgemäßen Verfahren einsetzbaren zirkulären DNA werden aus dem Plasmid des vorstehenden Typs pCLEAN4 solche DNA-Sequenzen entfernt, die die Replikation des Plasmids im Wirtsorganismus ermöglichen würden. Enthalten die Ausgangsvektoren mehrere DNA-Sequenzen, die beispielsweise für verschiedene Antibiotikum-Resistenzen codieren, so können auch die nicht benötigten Antibiotikum-Resistenz-DNA-Sequenzen aus dem Vektor entfernt werden. Im Falle des vorstehenden Beispiels des Plasmides pCLEAN4 sind beispielsweise die Funktionen repU und oriB sowie die Resistenzen gegen die Antibiotika Neomycin bzw. Kanamycin enthalten. Aufgrund der Kenntnisse der

3

Restriktionskarte lassen sich diese nicht benötigten DNA-Sequenzen aus dem Plasmid durch Schneiden mit geeignet ausgewählten Restriktionsendonukleasen entfernen. Im Falle des Plasmides pCLEAN4 wird beispielsweise mit einer Restriktionsendonuklease NsiI ein Fragment aus dem Vektor deletiert, welches die Funktionen für die Replikation des Plasmides in Bacillus-Spezies sowie die Resistenzen gegen Neomycin und Kanamycin enthält und anschließend die verbleibende DNA mit Hilfe der DNA-Ligase in eine zirkuläre DNA überführt. Die für ein interessierendes Polypeptid codierende DNA-Sequenz sowie die Sequenz für die Antibiotikum-Resistenz Phleomycin bleibt aus diesem deletierten Vektor als zirkuläre DNA (pCLEAN4-del) erhalten. Mit dieser zirkulären DNA ist mit Hilfe des interessierenden Polypeptid-Gens (hier eine hochalkalische Protease) eine Integration durch homologe Rekombination in das Chromosom von z.B. Bacillus alcalophilus möglich.

Die erfindungsgemäße zirkuläre DNA-Konstruktion kann auch hergstellt werden, indem man in vitro

a) die DNA-Sequenz, die für das interessierende Polypeptid codiert, und die z.B. durch Herausschneiden der entsprechenden DNA-Sequenz mit Restrikionsendonukleasen aus einem diese Sequenz enthaltenden Plasmid, mit

b) einem Resistenzmarker, der erhalten wurde, indem eine entsprechende DNA-Sequenz aus einem Plasmid, das diesen Resistenzmarker enthält, mit Restriktionsendonukleasen herausgeschnitten wurde, mit Hilfe von DNA-Ligase rekombiniert, so daß eine zirkuläre DNA entsteht.

Als Resistenzmarker eignet sich vorzugsweise jede für eine Antibiotikum-Resistenz codierende DNA-Sequenz, die

a) in dem zu transformierenden Mikroorganismus exprimiert wird und

b) die für die Resistenz gegen ein Antibiotikum codiert, gegen welches der zu transformierende Mikroorganismus nicht von Natur aus eine Resistenz aufweist.

Beispielsweise kann das Resistenzgen für eine Resistenz gegen ein Antibiotikum aus der Gruppe Chloramphenicol, Ampicillin, Tetracyclin, Kanamycin, Neomycin, Mitomycin C, Bleomycin oder Phleomycin codieren.

Besonders geeignet ist eine DNA-Sequenz aus dem Plasmid pUB110, die für die Resistenz gegen das Antibiotikum Phleomycin codiert.

Durch Transformation eines Mikroorganismus, der wenigstens bereits eine für ein interessierendes Polypeptid codierende DNA-Sequenz in der chromosomalen DNA enthält, kann über homologe Rekombination am Ort des Gens für das interessierende Polypeptid eine weitere für dieses Polypeptid codierende DNA-Sequenz mit den zugehörigen zur Expression und gegebenenfalls Sekretion benötigten Sequenzen in das Chromosom des Wirtsorganismus integriert werden. Hierbei wird die zusätzliche für ein interessierendes Polypeptid codierende DNA-Sequenz und die zugehörigen für die Expression und gegebenenfalls Sekretion benötigten Sequenzen durch einfaches Cross-Over derart in das Chromosom eingebaut, das zwischen der ursprünglich bereits enthaltenen für das Polypeptid codierenden DNA und der neu eingeführten ebenfalls für dieses Polypeptid codierenden DNA die für die Antibiotikum-Resistenz codierende DNA-Sequenz zu liegen kommt. Anschließend werden die transformierten Mikroorganismen mit Hilfe dieser Antibiotikum-Resistenz selektiert, indem man diese auf einem Nährmedium kultiviert, dem zusätzlich das entsprechende Antibiotikum in bestimmter Konzentration zugesetzt wurde. Unter diesen Bedingungen sind nur die Transformanten vermehrungsfähig, die die erfindungsgemäße zirkuläre DNA in ihre chromosomale DNA integriert haben. Die Konzentration des dem Kulturmedium zugesetzten Antibiotikums wird so gewählt, daß zumindest solche Transformanten selektiert werden, die wenigstens zwei für das interessierende Polypeptid codierende DNA-Sequenzen und eine für den Resistenzmarker codierende DNA-Sequenz in der chromosomalen DNA enthalten.

Durch Kultivieren derartiger Transformanten bei höheren Antibiotikum-Konzentrationen können solche Nachkommen isoliert werden, die mehrere Kopien der für die Antibiotiku-Resistenz codierenden DNA-Sequenz und somit auch mehrere Kopien der für das interessierende Polypeptid codierenden DNA-Sequenz im Genom aufweisen. Derartige Klone können sich durch weitere gesteigerte Synthese des interessierenden Polypeptides auszeichnen.

Bekannterweise erweisen sich derartige Konstruktionen mit benachbarten repetitiven DNA-Sequenzen im Genom in gängigen Laborstämmen als instabil. In industriellen Hochleistungsstämmen, die im allgemeinen nach mehrfachen Mutationszyklen isoliert wurden und die daher im allgemeinen ein vermindertes Rekombinationsvermögen aufweisen, welches z.B. durch erhöhte Sensibilität gegen das Antibiotikum Mitomycin C erkennbar ist, sind derartige repetitiven Sequenzen im Chromosom stabil genug, um eine großtechnische Produktion in Abwesenheit von Antibiotikum durchzuführen. Werden Instabilitäten beobachtet, so ist es möglich, rekombinationsdefizient Nachkommen zu isolieren, indem nach Mutation mit einem gängigen Mutagen nach solchen Mutanten gesucht wird, die eine erhöhte Sensibilität gegen das Antibiotikum Mitomycin C zeigen.

4

Erläuterungen zu den Figuren

Figur 1:     Restriktionskarte des Plasmids pCLEAN4
Figur 2:     Restriktionskarte des Plasmids pUB 131
Figur 3:     Restriktionskarte des Plasmids pLI 1
Figur 4:     Proteolytische Aktivitäten in den Kulturüberständen der Kulturen von B. alcalophilus HA 139/28 und HA 139/28-INT1

Beispiele

Die folgende Offenbarung gibt zur weiteren Erläuterung der Erfindung typische beispielhafte Ausgestaltungen der Erfindung wieder, ohne jedoch dadurch die Erfindung zu beschränken.

Sofern nicht anders angegeben, wurde generell nach Methoden gearbeitet, wie sie in Maniatis et al. (Maniatis et al. = T. Maniatis, E. F. Fritsch, J. Sambrook, Molecular Cloning, A Laboratory Manual, Cold Spring Harbor Laboratory, 1982) beschrieben sind.

Hier verwendete Ausgangsvektoren sind käuflich und auf unbeschränkter Basis verfügbar; oder sie können nach an sich bekannten Methoden aus verfügbaren Vektoren hergestellt werden. So wurde der Vektor M13g131 von Amersham, Buckinghamshire, England bezogen.

Die verschiedenen benutzten Restriktionsendonukleasen gehören zum Stand der Technik und sind kommerziell verfügbar. Die bei Verwendung dieser bekannten Restriktionsendonukleasen jeweils erforderlichen Reaktionsbedingungen sind ebenfalls bekannt.

Nach Inkubation mit einer Restriktionsendonuklease wurde das Protein auf an sich bekannte Weise durch Extraktion (z.B. mit Phenol und Chloroform) entfernt und die geschnittene DNA (z.B. aus der wäßrigen Fraktion durch Fällung mit Ethanol) isoliert und der weiteren Verwendung zugeführt.

An das Schneiden von Vektoren mit Restriktionsendonukleasen kann sich gegebenenfalls eine Hydrolyse des terminalen 5'-Phosphatrestes mit einer alkalischen Phosphatase (Dephosphorylierung) anschließen. Sofern in den Beispielen eine Dephosphorylierung des 5'-Endes vorgenommen wurde, geschah dieses in an sich bekannter Weise. Weitere Angaben zur Durchführung einer Dephosphorylierung und zu den dafür benötigten Reagentien können Maniatis et al. (S. 133 - 134) entnommen werden.

Partielle Hydrolyse bedeutet unvollständige Verdauung von DNA durch eine Restriktionsendonuklease. Die Reaktionsbedingungen werden dabei so gewählt, daß in einem DNA-Substrat zwar an einigen, nicht aber an allen Erkennungsstellen für die eingesetzte Restriktionsendonuklease geschnitten wird.

Zur Gewinnung und Isolierung von bestimmten DNA-Fragmenten, z.B. nach Behandlung von DNA mit Restriktionsendonukleasen, wurden die angefallenen DNA-Fragemente in an sich bekannter Weise durch Gelelektrophorese (z.B. auf Agarosegel) getrennt, nachfolgend über das Molekulargewicht (Bestimmung durch Vergleich mit Referenz-DNA-Fragmenten mit bekanntem Molekulargewicht) identifiziert und die gewünschten DNA-Fragmente aus den entsprechenden Gelzonen abgetrennt.

Ligationen können unter an sich bekannten Bedingungen, z.B. in einem Puffer mit etwa 10 Units T4-DNA-Ligase pro 0,5 $\mu$g einer etwa gleich molaren Menge der zu ligierenden DNA-Fragmente, ausgeführt werden (siehe z.B. Maniatis et al., S. 146).

Unter Transformation wird die Einschleusung von DNA in einen Mikroorganismus verstanden, so daß die DNA in diesem autonom als Plasmid-DNA repliziert oder chromosomal eingebaut wird und ggf. exprimiert werden kann. Für die Transformation von Bacillus-Spezies ist z.B. die von Anagnostopoulos et al. (1961, J. Bact. 81: 741 - 746) beschriebene Methode geeignet.

Eine Polyklonierungsstelle (Polylinker) ist ein kurzes bis mittleres doppelsträngiges DNA-Fragment, welches eng benachbart eine Vielzahl von Erkennungsstellen für Restriktionsendonukleasen aufweist. Eine in den Beispielen verwendete, aus dem Vektor M13tg131 entstammende Polyklonierungsstelle besitzt z.B. eine Größe von etwa 0,07 kB (Kilobasen) und weist Erkennungsstellen für 14 verschiedene Restriktionsendonukleasen auf.

Der im Beispiel 1 eingesetzte und als Bacillus alcalophilus HA 1 benannte Bacillus alcalophilus-Stamm ist bei der Deutschen Sammlung von Mikroorganismen (DSM) mit der DSM-Nummer 5466 am 28. Juli 1989 hinterlegt worden. Weitere verwendete Mikroorganismen sind käuflich verfügbar, z.B. Bacillus subtilis BD 244 (Bacillus Genetic Stock Center 1 A 46) oder Bacillus subtilis BD 366 (Bacillus Genetic Stock Center 1 E 6).

Weitere im Rahmen der Erfindung verwendete Mikroorganismen können aus dem Bacillus DSM 5466 bzw. aus käuflichen Mikroorganismen nach den nachfolgend angegebenen Verfahrensweisen erzeugt werden:

1. Bacillus alcalophilus HA 139/28 und HA 28/12

Beide Stämme sind Abkömmlinge vom Bacillus alcalophilus HA

1. Sie scheiden verstärkt eine hochalkalische Protease aus, wie diese z.B. in der deutschen Patentanmeldung 40 23 458 beschrieben ist, und können als industrielle Produktionsstämme benutzt werden. Derartige Stämme können erhalten werden, indem Bacillus alcalophilus HA 1 nach her kömmlichen und wohlbekannten Methoden mit mutagenen Substanzen, wie z.B. 1-Methyl-3-Nitro-1-Nitrosoguanidin behandelt wird und anschließend auf Protein-Agarplatten nach solchen Mutanten gesucht wird, die in der Lage sind, größere Hydrolysehöfe zu bilden als der Ausgangsstamm. Durch wiederholte Mutationszyklen lassen sich Stämme mit weiter gesteigerten Proteasebildung isolieren.

2. Bacillus licheniformis KC 14/1-422

Bei dem Stamm handelt es sich um einen Bacillus licheniformis, der verstärkt die alkalische Protease aussscheidet und als industrieller Produktionsstamm benutzt werden kann. Ein derartiger Stamm kann erhalten werden, indem z.B. ein Bacillus licheniformis Type Strain (beziehbar bei der Deutschen Sammlung für Mikroorganismen unter der Nummer DSM 13) nach herkömmlichen und wohlbekannten Methoden mit mutagenen Substanzen, wie z.B. 1-Methyl-3-Nitro-1-Nitrosoguanidin behandelt wird und anschließend auf Protein-Agarplatten nach solchen Mutanten gesucht wird, die in der Lage sind, größere Hydrolysehöfe zu bilden als der Ausgangsstamm. Durch wiederholte Mutationszyklen lassen sich Stämme mit weiter gesteigerter Proteasebildung isolieren.

3. Bacillus licheniformis SEETT 18

Bei dem Stamm handelt es sich um einen Bacillus licheniformis, der verstärkt $\alpha$-Amylase ausscheidet und als industrieller Produktionsstamm benutzt werden kann. Ein derartiger Stamm kann erhalten werden, indem z.B. ein Bacillus licheniformis Stamm (beziehbar bei der American Type Culture Collection unter der Nummer ATCC 27811) nach herkömmlichen und wohl-bekannten Methoden mit mutagenen Substanzen, wie z.B. 1-Methyl-3-Nitro-1-Nitrosoguanidin behandelt wird und anschließend auf Stärke-Agarplatten nach solchen Mutanten gesucht wird, die in der Lage sind, größere Hydrolysehöfe (detektiert nach Überschichten mit Lugolscher Lösung) zu bilden als der Ausgangsstamm. Durch wiederholte Mutationszyklen lassen sich Stämme mit weiter gesteigerter Amylasebildung isolieren.

Beispiel 1

Herstellung einer genomischen DNA-Bibliothek aus B. alcalophilus HA 1 und Isolierung des Gens für eine hochalkalische Protease

Aus dem Naturisolat Bacillus alcalophilus HA 1 (hinterlegt bei der Deutschen Sammlung von Mikroorganismen unter der Nummer DSM 5466) wurde nach der Methode von Saito et al. (1963, Biochim.Biophys.Acta. 72:619-629) chromosomale DNA isoliert und mit der Restriktionsendonuklease Sau3A partiell hydrolisiert.

Die Restriktionsfragmente wurden durch Elektrophorese auf einem Agarosegel aufgetrennt und die Fragmente mit einer Größe von 3 bis 8 kB wurden isoliert.

Die isolierten und größenselektierten DNA-Fragmente aus Bacillus alcalophilus HA 1 wurden mit Vektor-DNA des Plasmids pUB 110 (Gewinnung wie in Beispiel 2 beschrieben) in vitro neu kombiniert.

Hierzu wurde das Plasmid pUB110 zunächst mit der Restriktionsendonuklease BamH I restringiert und anschließend mit alkalischer Phosphatase aus Kälberdarm dephosphoryliert. Anschließend wurden 2 $\mu$g der restringierten und dephosphorylierten Vektor DNA mit 8 $\mu$g der Bacillus alcalophilus DNA-Fragmente in einem Gesamtvolumen von 100 $\mu$l mit T4-DNA Ligase 24 h bei 16 °C inkubiert.

Mit der erhaltenen in vitro neukombinierten DNA wurden Protoplasten des Stammes Bacillus subtilis BD 224 nach der von S. Chang und N. Cohen (1979, Mol. Gen. Genet. 168: 111 - 115) beschriebenen Methode transformiert. Die Transformanten wurden auf Platten mit Neomycin selektiert und anschließend auf Magermilchagar überführt. Unter 13800 untersuchten Transformanten wurde eine gefunden, die durch Proteolyse des Magermilchagars einen deutlich größeren Hof bildete. Aus diesem Klon wurde die Plasmid-DNA nach Maniatis et al. isoliert. Das in diesem Plasmid enthaltene klonierte Fragment aus der B. alcalophilus-DNA hatte eine Größe von 4,1 kB und enthielt die vollständige Information für die hochalkalische Protease aus Bacillus alcalophilus HA 1.

Zur Vereinfachung des weiteren Verfahrens wurde das 4,1 kB große DNA-Fragment zunächst in der Größe reduziert. Hierzu wurden die auf dem DNA-Fragment befindlichen Erkennungsstellen für Restriktionsendonukleasen durch Schneiden des Plasmids mit verschiedenen Restriktionsendonukleasen und Auftrennung der Fragmente der restringierten DNA durch Elektrophorese auf einem Agarosegel ermittelt. Es wurde ein 2,3 kB großes, durch Schneiden mit den Restriktionsendonukleasen Ava I und Hind III erhältliches DNA-

Fragment ermittelt, welches die vollständige Information für die hochalkalische Protease aufwies und welches für das weitere Verfahren verwendet wurde. Hierzu wurde das obige Plasmid mit dem 4,1 kB-Fragment mit den Restriktionsendonukleasen Ava I und Hind III restringiert. Das 2,3 kB große DNA-Fragment wurde isoliert und mit dem Vektor pUB 131 (Gewinnung wie in Beispiel 2 beschrieben), der zuvor ebenfalls mit Ava I und Hind III geschnitten wurde, ligiert.

Das erhaltene Plasmid, das die Bezeichnung pCLEAN4 erhielt, wurde in den Stamm B. subtilis BD 224 eingebracht. Die Transformanten waren in der Lage, die hochalkalische Protease auszuscheiden, was zeigt, daß das Ava I/Hind III-Fragment das vollständige Strukturgen für die hochalkalische Protease aus B. alcalophilus HA 1 enthält. Die Restriktionskarte des Plasmids pCLEAN4 ist in Fig. 1 wiedergegeben.

Beispiel 2

Isolierung und Reinigung des Plasmids pUB110 und Konstruktion des Vektors pUB 131

Aus dem Stamm Bacillus subtilis BD 366 (pUB 110) wurde nach der Methode von T.J. Gryczan et al. (1978,J.Bacteriol. 134: 318-329) das Plasmid pUB 110 isoliert und anschließend nach Maniatis et al. (S. 93) über Cäsiumchlorid-Dichtegradientenzentrifugation gereinigt. Der Vektor pUB 110 enthält nur einmal vorkommende Restriktionsstellen für die Restriktionsendonukleasen BamH I und EcoR I und als Marker DNA-Sequenzen, die für Antibiotika-Resistenzen gegenüber Neomycin und Phleomycin codieren, sowie für die Replikation in Bacillus-Spezies benötigte DNA-Sequenzen ("origin of replication").

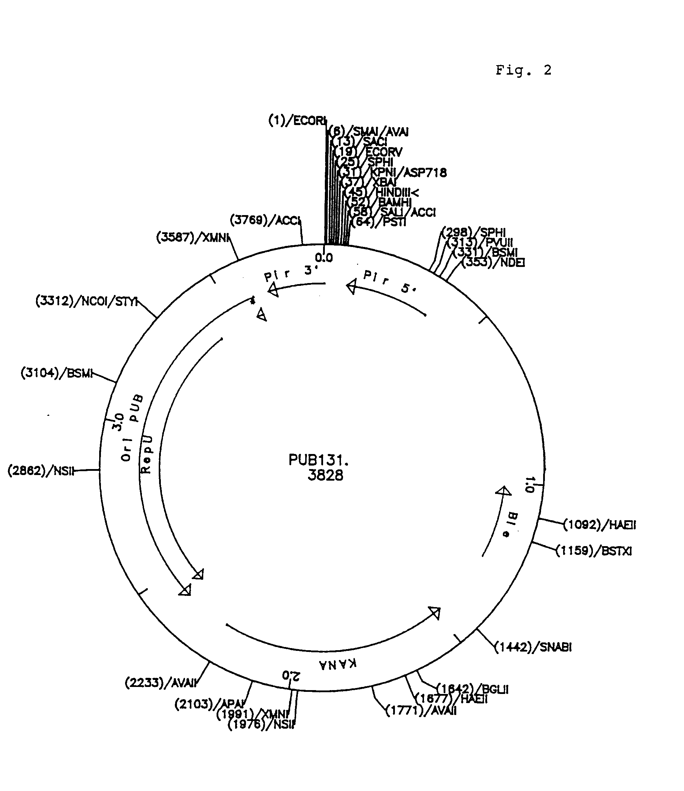

Das vorstehend erhaltene Plasmid pUB 110 wurde mit EcoR I und BamH I restringiert. Das kleinere Fragment mit 790 Bp (Basenpaare) wurde ersetzt durch einen aus 67 Basenpaaren bestehenden Polylinker, der zuvor als EcoR I/Bgl II-Fragment aus dem Vektor M13tg131 isoliert worden war. Der so erhaltene Vektor mit der Bezeichnung pUB 131 ist somit ein Abkömmling von pUB 110, bei dem das etwa 0,8 kB große EcoR I/BamH I Fragment deletiert und dafür eine Polyklonierungsstelle eingebaut wurde. Die Restriktionskarte des Vektors mit der Bezeichnung pUB 131 ist in Fig. 2 wiedergegeben.

Beispiel 3

Transformation von B. alcalophilus-Spezies

B. alcalophilus HA 139/28 und HA 28/12 wurden nach der von Chang und Cohen (1979, Mol. Gen. Genet. 168: 111-115) beschriebenen Protoplasten-Methode transformiert, die wie folgt modifiziert war:
Das Wuchsmedium enthielt 1 % 1 molaren Carbonatpuffer (53 g $Na_2CO_3$, 42 g $NaH_2CO_3$ ad 1000 ml). Der pH-Wert der "DM3"-Regenerationsplatten wurde vor dem Gießen mit NaOH auf pH 7,8 eingestellt. Die Regenerationsplatten enthielten zur Selektion der Transformanten 30 $\mu$g/ml Phleomycin.

Beispiel 4

Chromosomale Genamplifikation der hochalkalischen Protease aus B. alcalophilus HA 1

Wie die Restriktionskarte des Vektors pUB 131 in Fig. 2 zeigt, befindet sich bei 1976 Bp und 2862 Bp jeweils eine Schnittstelle für die Restriktionsendonuklease Nsi I. Durch Entfernung des dazwischen liegenden 886 Bp-Fragmentes können die Funktionen repU, oriB und neo[r]/kana[r] somit deletiert werden, so daß die resultierende DNA nicht mehr in der Lage ist, sich in einer Wirtszelle autonom zu replizieren. Das Plasmid pCLEAN4 (siehe Fig. 1) enthält im 2,3 kB großen DNA-Fragment für die hochalkalische Protease aus B. alcalophilus HA 1 keine Erkennungsstellen für Nsi I. Durch Deletion des 886 Bp-Nsi I-Fragmentes aus dem Plasmid pCLEAN4 erhielt man eine DNA, die in einer Wirtszelle nicht mehr autonom replizierbar war, mit der jedoch B. alcalophilus und andere Bacilli transformiert werden konnten, da

- diese DNA weiterhin den Phleomycinresistenzmarker enthielt und somit selektierbar war und
- mit Hilfe des Protease-Gens eine Integration über homologe Rekombination in das Chromosom von z.B. B. alcalophilus möglich war.

10 $\mu$g Plasmid pCLEAN4 wurden mit Nsi I geschnitten. Die hydrolisierte DNA wurde auf einem Agarosegel aufgetrennt und das größere Fragment (5173 Bp) wurden isoliert. Dieses wurde mit Hilfe der T4-DNA-Ligase in zirkuläre DNA überführt.

Diese zirkuläre DNA mit der Bezeichnung pCLEAN4-del codierte nicht mehr für die Resistenz gegen das Antibiotikum Neomycin/Kanamycin und konnte, da die Funktionen repU und oriB fehlten, nicht mehr autonom repliziert werden bzw. somit in Bakterienzellen nicht mehr als Plasmid vorliegen.

Die zirkuläre DNA pCLEAN4-del wurde nachfolgend in Protoplasten der Stämme B. alcalophilus HA 28/12 und B. alcalophilus HA 139/28 eingebracht. Die Selektion erfolgte nach Phleomycinresistenz (siehe Beispiel 3).

Unter den erhaltenen Transformanten wurde beim Stamm HA 28/12 die Transformante HA 28/12-INT 542 und beim Stamm HA 139/28 die Transformante HA 139/28-INT 1 für weitere Untersuchungen ausgewählt. Bei beiden Transformanten konnte auch bei mehrfach versuchter Plasmidisolation keine Plasmid-DNA nachgewiesen werden. Mit der aus diesen Transformanten isolierten DNA konnte B. subtilis nicht nach Phleomycin- oder Kanamycin/Neomycinresistenz transformiert werden. Es war somit sichergestellt, daß die Transformanten frei von episomalen Elementen waren.

Per Southern Blotting konnte gezeigt werden, daß die pCLEAN4-del-DNA über homologe Rekombination am Ort des Gens für die hochalkalische Protease in das Chromosom des jeweiligen Wirtsorganismus HA 28/12 bzw. HA 139/28 integriert worden war.

Die Stämme B.alcalophilus HA 139/28-INT 1 und B.alcalophilus HA 28/12-INT 542 wurden auf "DM3"-Regenerationsplatten, die 30 $\mu$g/ml Phleomycin enthielten, isoliert. Das Ausmaß der Resistenz gegen Phleomycin war abhängig von dem verwendeten Wuchsmedium. Die oben genannten Transformanten waren auf üblichen Labormedien wie z.B. Tryptose Blood Agar Base Platten oder Luria Broth Agar Platten nur gegen eine Phleomycin-Konzentration von maximal 3 $\mu$g/ml resistent. Eine Adaption an höhere Phleomycin-Konzentration war möglich durch wiederholtes Kultivieren der Stämme in Flüssigmedien mit steigenden Konzentrationen an Phleomycin. Derartig adaptierte Stämme enthielten pro Genom mehrere Kopien des Phleomycinresistenz-Gens sowie mehrere Kopien des Gens für die hochalkalische Protease aus B.alcalophilus HA 1.

Beispiel 5

Bestimmung der Protease-Ausbeuten in Schüttelkolben

Die Stämme B. alcalophilus HA 139/28-INT 1 und B. alcalophilus HA 28/12-INT 542 aus Beispiel 4, die entsprechenden Ausgangsstämme und Stämme, die an höhere Phleomycin Konzentrationen adaptiert worden waren (Beispiel 4), wurden unter den im folgenden beschriebenen Bedingungen kultiviert. Nach einer Kulturdauer von 31 h wurden die proteolytischen Aktivitäten in den Kulturüberständen gemessen. Die Ergebnisse sind in der Tabelle 1 wiedergegeben. Die Ausbeute des Ausgangsstammes wurde jeweils als 100 % eingesetzt.

Kulturbedingungen:

50 ml Vorkulturmedium (15 g Tryptone, 7,5 g Hefe Extrakt, 5 g NaCl, 50 g Stärke, 10 ml Maisquellwasser in 1000 ml) wurden mit einer Einzelkolonie des zu testenden Stammes beimpft und für 18 h bei 37 °C und 320 Upm in Erlenmeyerkolben mit Schikanen inkubiert. Mit 2,5 ml dieser Kultur wurden 50 ml Hauptkulturmedium (40 g Sojaschrot, 100 g Stärke, 20 ml Maisquellwasser in 1000 ml) beimpft und bei 37 °C und 320 Upm in Erlenmeyerkolben mit Schikanen inkubiert. Beide Medien enthielten zusätzlich 150 ml Carbonatpuffer (53 g $Na_2Co_3$, 42 g $NaHCO_3$ in 1000 ml) pro 1000 ml Kulturmedium. Die Ergebnisse sind in Tabelle 1 wiedergegeben.

Tabelle 1

| Stamm B.alcalophilus | resistent gegen Phleomycin | Aktivität im Kulturüberstand |
|---|---|---|
| HA 139/28 | 0 $\mu$g/ml | 100 % |
| HA 139/28-INT 1 | 3 $\mu$g/ml | 170 % |
| HA 139/28-INT 1 | 5 $\mu$g/ml | 175 % |
| HA 139/28-INT 1 | 10 $\mu$g/ml | 180 % |
| HA 139/28-INT 1 | 20 $\mu$g/ml | 160 % |
| HA 139/28-INT 1 | 25 $\mu$g/ml | 120 % |
| HA 28/12 | 0 $\mu$g/ml | 100 % |
| HA 28/12-INT 542 | 3 $\mu$g/ml | 135 % |
| HA 28/12-INT 542 | 10 $\mu$g/ml | 135 % |

Beispiel 6

Stabilität der DNA-Integrationen

a) B.alcalophilus HA 28/12-INT 542 aus Beispiel 4 wurde für 50 Generationen in Luria-Broth (10 g Tryptone, 5 g Hefe Extrakt, 5 g NaCl in 1000 ml $H_2O$ - 10 ml Carbonatpuffer / 1 l Medium) kultiviert. Anschließend wurden die Bakterien auf Agar-Platten ohne Antibiotika vereinzelt und nach Bebrütung der Petrischalen wurden 100 Kolonien auf AgarPlatten, die 3 $\mu$g/ml Phleomycin enthielten, überführt. Es wurde keine Kolonie gefunden, die in der Gegenwart von Phleomycin nicht zu wachsen vermochte. Das Ergebnis zeigt, daß der Marker Phleomycinresistenz im Chromosom eine hohe Stabilität aufweist.

b) Die Stämme B. alcalophilus HA 139/28 und B. alcalophilus HA 139/28-INT 1 aus Beispiel 4 wurden wie im Beispiel 5 (Medienrezeptur und Kulturbedingungen) beschrieben kultiviert.

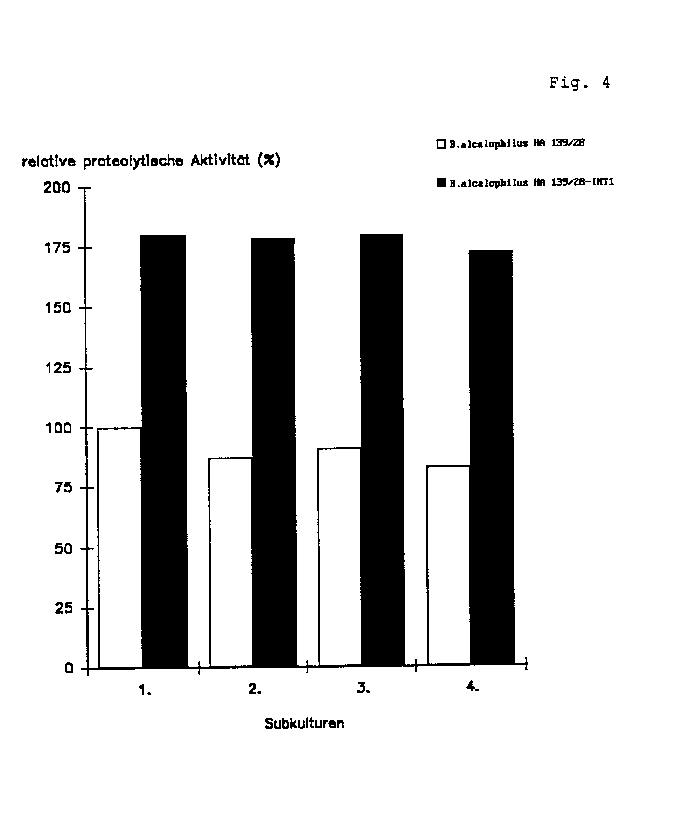

20 ml Vorkulturmedium wurden jeweils mit einer Einzelkolonie der Stämme von einer Agar-Platte beimpft. Das Medium für B.alcalophilus HA 139/28-INT 1 enthielt 10 $\mu$g/ml Phleomycin (der Stamm war zuvor an die entsprechende Antibiotika-Konzentration adaptiert). Nach einer Inkubationsdauer von 18 h wurden 50 $\mu$l dieser Kultur in 50 ml Hauptkulturmedium ohne Phleomycin überführt. Nach einer Inkubationsdauer von 24 Stunden wurden die Zellen mit einer Verdünnungsrate von 1:1000 in frisches Hauptkulturmedium überführt. Es wurden insgesamt vier derartige Subkulturen in Abwesenheit von Phleomycin durchgeführt. Die proteolytischen Aktivitäten in den Kulturüberständen der einzelnen Kulturen wurden jeweils nach 31 h bestimmt. In der Figur 4 ist das Ergebnis dieses Versuches dargestellt. Die proteolytische Aktivität in der ersten Kultur von B.alcalophilus HA 139/28 wurde als 100 % gesetzt. Das Experiment zeigt, daß der Phänotyp "verstärkte Protease-Ausscheidung" von B. alcalophilus HA 139/28-INT 1 äußerst stabil ist.

Beispiel 7

Chromosomale Genamplifikation von alkalischer Protease aus Bacillus licheniformis

Es wurde eine chromosomale Amplifikation des Gens für die alkalische Protease von B. licheniformis in einem proteaseüberausscheidenden B. licheniformis durchgeführt. Die Experimente wurden entsprechend denen für die Amplifikation des Gens für die hochalkalische Protease von B. alcalophilus durchgeführt (siehe Beispiele 3 bis 5).

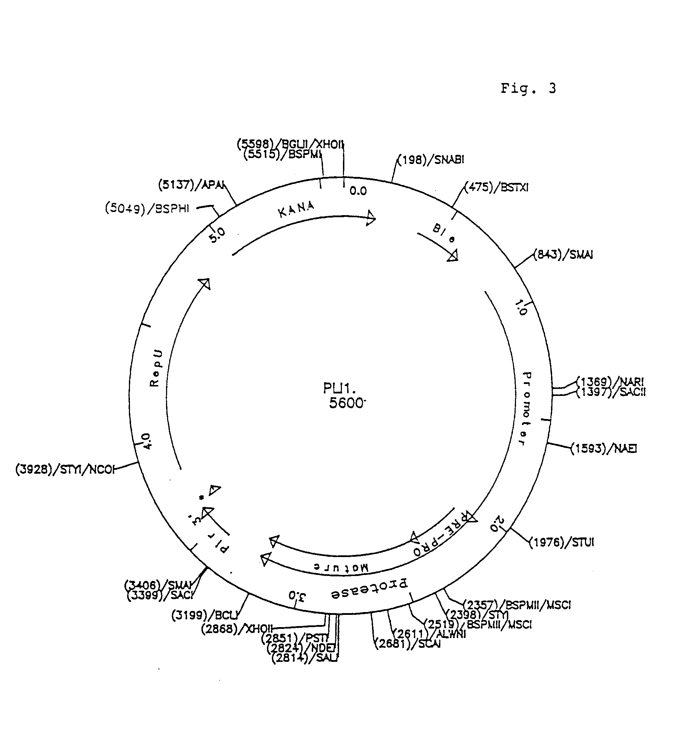

Figur 3 zeigt die Restriktionskarte vom Plasmid pLI 1. Das Plasmid ist ähnlich wie das Plasmid pCLEAN4 aufgebaut, das dem 2,3 kB großen DNA-Fragment in pCLEAN4 entsprechende Insert codiert, jedoch für die alkalische Protease aus B. licheniformis anstatt für die hochalkalischen Protease aus B. alcalophilus wie bei pCLEAN4.

Die Deletion der DNA-Sequenzen, die für die autonome Replikation benötigt werden, mit Hilfe der Restriktionsendonuklease NsiI war beim Plasmid pLI 1 nicht möglich, weil eine weitere Erkennungsstelle für Nsi I in der DNA Sequenz liegt, die für die alkalische Protease codiert.

Plasmid pLI 1 wurde daher mit den Restriktionsendonukleasen BspH1 und Nco 1 restringiert und über Agarosegelelektrophorese wurde das 4479 kB große Fragment isoliert, welches anschließend mit Hilfe der T4-DNA-Ligase in zirkuläre DNA überführt wurde. Die Ligation war möglich, da beide Nukleasen die gleichen überstehenden Einzelstränge erzeugen. Die zirkuläre DNA wurde per Protoplasten-Transformation in den Stamm B. licheniformis KC 14/1-422 eingebracht. Die Transformation wurde wie bei Chang und Cohen (1979, Mol. Gen. Genet. 168: 111 - 115) beschrieben durchgeführt. Die Selektion erfolgte auf "DM3"-Regenerationsplatten, die 35 $\mu$g/ml Phleomycin enthielten. Unter den erhaltenen Transformanten wurde der Stamm B. licheniformis KC 14/1-422-INT 14 ausgewählt.

B. licheniformis KC 14/1-422 INT 14 und B. licheniformis KC 14/1-422 wurden wie im Beispiel 5 beschrieben kultiviert.

Die Medien enthielten jedoch keinen Carbonatpuffer. Die proteolytische Aktivität im Kulturüberstand von B. licheniformis KC 14/1-422-INT 14 war um 20 % höher als bei B. licheniformis KC 14/1-422.

Beispiel 8

Chromosomale Genamplifikation von $\alpha$-Amylase aus Bacillus licheniformis

Es wurde eine chromosomale Amplifikation des Gens für die alpha-Amylase von B. licheniformis in einem Amylase überausscheidenden B. licheniformis beschrieben.

Die Experimente wurden entsprechend denen für die Amplifikation des Gens für die hochalkalische Protease von B. alcalophilus durchgeführt (siehe Beispiele 3 bis 5).

Das Plasmid pSEH 1 ist ähnlich wie das Plasmid pCLEAN4 aufgebaut, das der Protease in pCLEAN4 hier entsprechende Insert codierte jedoch für die α-Amylase aus B. licheniformis ATCC 27811.

Die Deletion der DNA-Sequenzen im Plasmid pSEH 1, die für die autonome Replikation benötigt werden, wurde mit Hilfe der Restriktionsendonuklease Nsi I durchgeführt. Die zirkuläre DNA wurde per Protoplasten-Transformation in den Stamm B. licheniformis SEETT 18 eingebracht. Unter den erhaltenen Transformanten wurde der Stamm B. licheniformis SEETT 18-INT 13 ausgewählt. B. licheniformis SEETT 18-INT 13 und B. licheniformis SEETT 18 wurden in einem Medium, das Lactose, Baumwollsamenmehl, Sojamehl und Maisquellwasser enthielt kultiviert. Die amylolytische Aktivität im Kulturüberstand von B. licheniformis SEETT 18-INT 13 war um 25 % höher als bei B. licheniformis SEETT 18.

**Patentansprüche**

1. Verfahren zur Herstellung einer transformierten prokaryotischen Wirtszelle, die die für ein interessierendes Polypeptid codierende DNA-Sequenz wenigstens zweifach chromosomal enthält, dadurch gekennzeichnet, daß man

   eine prokaryotische Wirtszelle, die wenigstens bereits eine für ein interessierendes Polypeptid codierende DNA-Sequenz in der chromosomalen DNA enthält,

   mit einer zur Transformation der Wirtszelle geeigneten zirkulären DNA aus im wesentlichen

   a) einem DNA-Fragment, welches die für ein interessierendes Polypeptid codierende DNA-Sequenz einschließlich der für die Expression und gegebenenfalls für die Sekretion des interessierenden Polypeptids benötigten Sequenzen enthält, und aus

   b) wenigstens einem DNA-Fragment mit einer DNA-Sequenz, die für einen selektierbaren Marker codiert,

   wobei diese zirkuläre DNA keine DNA-Sequenz enthält, die sie zur autonomen Replikation in der Wirtszelle befähigt, transformiert,

   anschließend mit Hilfe des selektierbaren Markers solche Zellen, die die verwendete zirkuläre DNA wenigstens einmal chromosomal integriert haben, selektiert und die derart transformierten Zellen isoliert.

2. Verfahren nach Anspruch 1, dadurch gekennzeichnet, daß als Wirtszellen Bacilli, vorzugsweise Bacillus alcalophilus oder Bacillus licheniformis eingesetzt werden.

3. Verfahren nach Anspruch 1, dadurch gekennzeichnet, daß die für ein interessierendes Polypeptid codierende DNA-Sequenz in Fragment a) für eine Protease, vorzugsweise eine alkalische Protease, codiert.

4. Verfahren nach Anspruch 1, dadurch gekennzeichnet, daß die für ein interessierendes Polypeptid codierende DNA-Sequenz in Fragment a) für eine Amylase, vorzugsweise eine α-Amylase, codiert.

5. Verfahren nach Anspruch 1, dadurch gekennzeichnet, daß als selektierbare Marker in Fragment b) eine DNA-Sequenz eingesetzt wird, die für die Resistenz gegen ein Antibiotikum codiert.

6. Verfahren nach Anspruch 5, dadurch gekennzeichnet, daß als selektierbarer Marker in Fragment b) eine DNA-Sequenz eingesetzt wird, die für die Resistenz gegen das Antibiotikum Phleomycin codiert.

Fig. 1

(1)/ECORI
(6)/AVAI
(106)/PVUI
(153)/MLUI
(539)/MLUI
(755)/HPAI
(929)/PVUI
(1207)/NCOI
(1235)/PVUII
(1242)/BSTXI
(1618)/XBAI
(1650)/ECORI
(2224)/ASP718/KPNI
(2276)/HINDIII<
(2283)/BAMHI
(2289)/SALI
(2295)/PSTI
(2544)/PVUII
(2584)/NDEI
(3390)/BSTXI
(3673)/SNABI
(3873)/BGLI
(4207)/NSII
(4334)/APAI
(4336)/BGLI
(5093)/NSII
(5543)/NCOI

(5543)/NCOI

Pir 3'
Ori PUB
RepU
KANA
Bla
Pir 5'
Mature
Protease

PCLEAN4.
6059

6.0
0.0
1.0
2.0
3.0
4.0
5.0

11

Fig. 2

Fig. 3

Fig. 4

relative proteolytische Aktivität (%)

☐ B.alcalophilus HA 139/28

■ B.alcalophilus HA 139/28-INT1

Subkulturen